# EUROPÄISCHE PATENTSCHRIFT

(11) **EP 2 789 367 B1**
(45) Veröffentlichungstag und Bekanntmachung des Hinweises auf die Patenterteilung: **25.10.2017**
(21) Anmeldenummer: 14162988.1
(22) Anmeldetag: 01.04.2014
(51) Int. Cl.: A61N 1/05, A61B 5/042

(54) **Kontaktierungseinrichtung für elektrische Verbindungen an flexiblen Elektrodenleitungen**
Contacting device for electrical connections to flexible electrode lines
Dispositif de contact pour des connexions électriques sur des lignes d'électrode flexibles

(30) Priorität: 09.04.2013 US 201361809896 P
(43) Veröffentlichungstag der Anmeldung: 15.10.2014
(73) Patentinhaber: BIOTRONIK SE & Co. KG, 12359 Berlin (DE)
(72) Erfinder: Jadwizak, Detmar, 15537 Erkner (DE); Weitzig, Pierre, 54666 Irrel (DE); Palm, Jochen, 15831 Mahlow (DE); Fründt, Carsten, 13057 Berlin (DE); Hillebrand, Gordon, 12157 Berlin (DE)
(74) Vertreter: Galander, Marcus

(56) Entgegenhaltungen:
- EP-A2- 1 754 508
- DE-A1-102005 039 038
- US-A- 4 711 027
- US-A1- 2003 236 562

## Beschreibung

Die Erfindung betrifft eine Kontaktierungseinrichtung für elektrische Verbindungen an flexiblen, in einen Patientenkörper einführbaren, insbesondere implantierbaren Elektrodenleitungen. Bei derartigen Elektrodenleitungen kann es sich um Dauerimplantate, wie kardiale Elektrodenleitungen oder Neuro-Elektroden, aber auch nur zeitweise in den Patientenkörper eingeführte Elektrodenleitungen handeln, wie beispielsweise Katheter in Form von Ablationskathetern.

Derartige Elektrodenleitungen weisen bekanntermaßen einen elastischen, langgestreckten, schlauchförmigen Elektrodenkörper auf, in dem eine Leitungswendel mit mehreren, koradial gebündelten Wendeldrähten von einer entsprechenden Basiseinheit, wie beispielsweise einem implantierten Herzschrittmacher, zu den jeweils vorgesehenen Elektroden, wie einer Ring- und einer Kopf-Elektrode geführt sind.

Ein grundsätzliches Problem stellt bei einer derartigen Elektrodenleitung die zugsichere, also mechanisch, und elektrisch sichere elektrische Verbindung zwischen der Leitungswendel und der jeweiligen Elektrode dar. Hier müssen geeignete Zugentlastungsmaßnahmen vorgesehen sein, insbesondere um die dortige mechanische und elektrische Verbindung zwischen der Leitungswendel und der jeweiligen Elektrode vor den dynamischen Dauerbelastungen zu schützen.

Aus dem Stand der Technik sind verschiedene konstruktive Maßnahmen für diese Problematik bekannt. So laufen zu kontaktierende Drähte oder Seile, die die Leitungswendel bilden, axial zwischen zwei Kontakthülsen durch und werden zwischen einer Innen- und Außenhülse geklemmt. Hierbei können die Bauteile miteinander vercrimpt oder auch widerstands- oder lasergeschweißt werden.

Eine andere bekannte Konstruktionslösung sieht vor, eine zu kontaktierende Wendel im Verbund auf einen ringschulterförmigen Absatz einer die Elektrode bildenden Hülse aufzusetzen und dort mittels Schweißen elektrisch anzubinden und mechanisch zu koppeln.

Umgekehrt ist es auch üblich, eine zu kontaktierende Wendel in eine Elektrodenhülse zur Bildung der Kopf- bzw. Ringelektrode einzuschieben und dort mittels Widerstands- oder Laserschweißen mechanisch und elektrisch anzubinden.

Schließlich ist es auch bekannt, ein zu kontaktierendes Seil axial in eine Aussparung in einer Ringhülse einlaufen zu lassen, wo durch Schweißen oder Crimpen eine Verbindung hergestellt werden kann.

Alle vorgenannten Verbindungstechniken, wie sie durch offenkundige Vorbenutzungen bekannt sind, zeigen den Nachteil, dass bei auftretenden dynamischen Bewegungen, Dehnungen und Zugbeanspruchungen eine direkte Belastung auf die Verbindung bzw. auf den Übergang vom flexiblen Zuleitungsbereich auf den starren, zu kontaktierenden Bereich vorhanden ist. Dies setzt eine hohe Dauerfestigkeit dieser herkömmlichen, elektrischen und mechanischen Verbindungen voraus. Insbesondere bei immer kleiner werdenden Bauteilen mit geringeren Querschnitten kann die erforderliche Sicherheit für eine Kraftübertragung und eine elektrische Kontaktierung oft nicht mehr erfüllt werden. Ein weiteres Problem ist, dass die thermischen Verfahren, wie Schweißverfahren, Prozessschwankungen unterliegen und zudem eine mechanische Schwächung des anzubindenden Drahtes verursacht. Deshalb ist es aus Sicherheitsgründen bis jetzt nicht möglich, z.B. einen einzelnen Wendeldraht mit Kontakt-Ringhülsen oder Kopfelektroden zu verbinden, wobei gleichzeitig eine ausreichende Zugkraftübertragung realisiert ist.

Eine Lösung dieses Problems ist aus der Patentanmeldung EP 1 54 508 A2 bekannt, welche die bekannte implantierbare Elektrodenleitung dadurch verbessert, dass sie eine innere Fixier-Hülse mit einem axial verlaufenden Durchführungsschlitz für ein aus der Leitungswendel herausgeführten Wendeldraht-Teilbündel, eine in Peripherrichtung umlaufende Wickelnut für dieses herausgeführte Teilbündel aufweist. Zwischen der Fixier-Hülse und dem geführten Wendeldraht-Teilbündel existiert eine zugsichere Fixierung, welche durch eine Umschlingung des herausgeführten Wendeldraht-Teilbündels um die Fixier-Hülse in der genannten Wickelnut um einen Mindest-Umschlingungswinkel realisiert ist. Auf der genannten inneren Fixierhülse sitzt eine mit dem herausgeführten Wendeldraht-Teilbündel elektrisch kontaktierte Elektroden-Hülse.

Ausgehend von der geschilderten Problematik des Standes der Technik liegt der Erfindung die Aufgabe zugrunde, eine Kontaktierungseinrichtung für elektrische Verbindungen an den geschilderten Elektrodenleitungen zu schaffen, die eine zuverlässige Zugentlastung der elektrischen Verbindung zwischen Leitungswendel und entsprechender Elektrode mit optimiertem Schutz vor Zug- und/oder anderer mechanischer/dynamischer Dauerbelastung sowie eine weitere Stabilisierung der zugsicheren Fixierung der Leitungswendel bietet.

Diese Aufgabe wird durch die angegebenen Merkmale des Anspruches 1 gelöst, wonach eine Kontaktierungseinrichtung für elektrische Verbindungen an flexiblen, in einen Patientenkörper einführbaren, insbesondere implantierbaren Elektrodenleitungen vorgesehen ist, welche umfasst:
- eine Leitungswendel (1) mit mehreren, koradial gebündelten Wendeldrähten,
- eine innere Fixier-Hülse für ein Teilbündel der Wendeldrähte, wobei die Fixier-Hülse einen in axialer Richtung verlaufenden Durchführungsschlitz für das Teilbündel der Wendeldrähte, eine in Peripherrichtung umlaufende Wickelnut für das herausgeführte Teilbündel sowie eine Freimachung am Durchführungsschlitz zum Hindurchführen des Teilbündels aufweist,
- eine äußere, auf der inneren Fixierhülse sitzenden, mit dem Teilbündel elektrisch kontaktierte Elektroden-Hülse und
- eine zugsichere Fixierung zwischen der Fixier-Hülse und dem durch den Durchführungsschlitz bzw. die Freimachung geführten Teilbündel durch eine Umschlingung des herausgeführten Teilbündels um die Fixier-Hülse in der Wickelnut um einen Mindest-Umschlingungswinkel.

Die Kontaktierungseinheit zeichnet sich dadurch aus, dass der Durchführungsschlitz in axialer Richtung durchgehend in der Fixierhülse angelegt und zur zusätzlichen zugsicheren Fixierung zwischen der Fixier-Hülse und dem Teilbündel die Fixier-Hülse auf die Leitungswendel unter plastischer Verformung aufgepresst ist.

Aufgrund dieser erfindungsgemäßen Kontaktierung sind die mechanischen Anforderungen an die Kontaktverbindung wegen der wirksamen Zugentlastung deutlich verringert. Diese Zugentlastung wird in erster Linie durch die Umschlingung des aus mindestens einem Wendeldraht bestehenden, aus der Leitungswendel herausgeführten und um die Fixierhülse herumgewickelten Teilbündels um die Fixierhülse geschaffen. Dadurch wird die Sicherheit der Kontaktierung insbesondere bei Einzeldrahtverbindungen - das Teilbündel umfasst dann nur einen einzigen herausgeführten Wendeldraht - erheblich erhöht.

In den abhängigen Ansprüchen sind bevorzugte Weiterbildungen der Erfindung angegeben. So kann die Wendel eine an sich bekannte Vierfach- oder Achtfach-Koradial-Wendel sein. Die Erfindung ist gleichermaßen an beiden Wendeltypen ohne Abstriche einsetzbar. Vorzugsweise umfasst das aus der Wendel herausgeführte Teilbündel zwei benachbarte Wendeldrähte.

Versuche bei der Entwicklung der Erfindung haben gezeigt, dass eine zuverlässige mechanische und elektrisch kontaktierende Verbindung bereits durch eine relativ geringe Umschlingung des herausgeführten Teilbündels um die Fixier-Hülse in deren Wickelnut insbesondere bei einer Wicklung des Drahtes in die Wickelnut unter radialer Vorspannung gewährleistet werden kann. Die Fixierung erfolgt nämlich durch reibungsbedingte Selbsthemmung der Drähte. Der Mindest-Umschlingungswinkel des Teilbündels um die Fixier-Hülse beträgt dabei mindestens 60°, bevorzugt mindestens 70°.

Eine weitere Stabilisierung der zugsicheren Fixierung der Leitungswendel kann durch eine bevorzugte Weiterbildung erreicht werden, bei der der Durchführungsschlitz in axialer Richtung durchgehend in der Fixierhülse angelegt und zur zusätzlichen zugsicheren Fixierung zwischen der Fixier-Hülse und dem Teilbündel die Fixier-Hülse auf die Leitungswendel unter plastischer Verformung aufgepresst ist.

Falls Wendeldrähte mit einer Isolierung versehen sind, ist für eine zuverlässige Kontaktierung die Entfernung der Isolierung im Bereich des herausgeführten Teilbündels eine bevorzugte Maßnahme.

Zur weiteren Stabilisierung der mechanischen und elektrischen Verbindung zwischen dem herausgeführten Teilbündel der Leitungswendel und der Fixier-Hülse kann ein stoffschlüssiges Fügeverfahren, wie insbesondere eine Verschweißung, zwischen diesen Bauteilen vorgesehen sein. Auch eine Verklebung insbesondere mit einem elektrisch leitfähigen Klebstoff ist denkbar.

Die erfindungsgemäße Kontaktierungsvorrichtung kann in unterschiedlichen Ausführungsformen zur Kontaktierung einer Ring- oder Kopfelektrode verwendet werden. Im erstgenannten Fall kann die nach dem Herausführen des Teilbündels verbleibende Restwendel über die Fixier-Hülse hinaus weitergeführt und die Elektroden-Hülse über die Restwendel hinweg auf die Fixier-Hülse aufgeschoben und dort fixiert werden. Dies ist eine konstruktiv einfache Realisierungsmöglichkeit für die Kontaktierung und mechanische Anbindung einer Ring-Elektrode an die Leitungswendel.

Bei der Kontaktierung einer Kopfelektrode endet vorzugsweise die nach dem Herausführen des Teilbündels verbleibende Restwendel in der Fixier-Hülse, wobei die Elektroden-Hülse einfach auf die Fixier-Hülse aufgeschoben und dort fixiert ist.

Um die Wickelnut in der Fixier-Hülse möglichst optimal an den Wendeldraht des herausgeführten Teilbündels anpassen zu können, entspricht die radiale Tiefe der Wickelnut vorzugsweise zumindest dem Durchmesser des Wendeldrahtes. Die axiale Länge der Wickelnut muss so groß sein, dass alle Drähte des Teilbündels hineinpassen. Es ist also mindestens der Durchmesser eines Wendeldrahtes vorzusehen, vorzugsweise ein ganzzahliges Vielfaches davon.

Die Vorteile der erfindungsgemäßen Lösung lassen sich wie folgt zusammenfassen:
- Eine elektrische Verbindung zwischen einer Leitungswendel und den entsprechenden Elektroden einer in den Körper einführbaren, flexiblen Elektrodenleitung wird vor Zug- und/oder mechanischer Dauerbelastung wirkungsvoll geschützt.
- Die mechanischen Anforderungen an die elektrische Kontaktierung werden verringert.
- Es ergibt sich eine vielfach größere Sicherheit für den Bestand der elektrischen und mechanischen Verbindung eines Dauerimplantates über die gesamte Lebensdauer.
- Fertigungsbegleitende mechanische Prüfungen, wie Zugproben können wegfallen oder werden zumindest stark reduziert.
- Es ist möglich, eine Einzeldrahtverbindung zu realisieren, die auch für die Zugkraftübertragung verantwortlich ist.
- Auch schwer oder nicht schweißbare Materialkombinationen können verwendet werden, wobei dann die bisher verwendeten, aufwändigen und platzraubenden Crimpverfahren nicht mehr zum Einsatz kommen müssen.
- Drähte können auch durch andere Verbindungsverfahren, wie beispielsweise Löten, kontaktiert werden, da die mechanische Kraftübertragung durch die erfindungsgemäße Zugentlastung erfolgt.

Weitere Merkmale, Einzelheiten und Vorteile der Erfindung ergeben sich aus der nachfolgenden Beschreibung von bevorzugten Ausführungsbeispielen anhand der beigefügten Zeichnungen. Es zeigen:
- Fig. 1: eine perspektivische Teildarstellung einer Leitungswendel im Bereich einer zu kontaktierenden Ringelektrode in einem Fertigungszwischenschritt,
- Fig. 2: eine perspektivische Darstellung der dabei verwendeten Fixier-Hülse,
- Fig. 3: eine perspektivische Teildarstellung analog Fig. 1 im Endmontagezustand einer Ringelektrode,
- Fig. 4: eine perspektivische Teildarstellung einer Leitungswendel im Bereich einer Kopfelektrode in einem Fertigungszwischenschritt, und
- Fig. 5: eine perspektivische Teildarstellung analog Fig. 4 in Endmontagezustand einer Kopfelektrode.

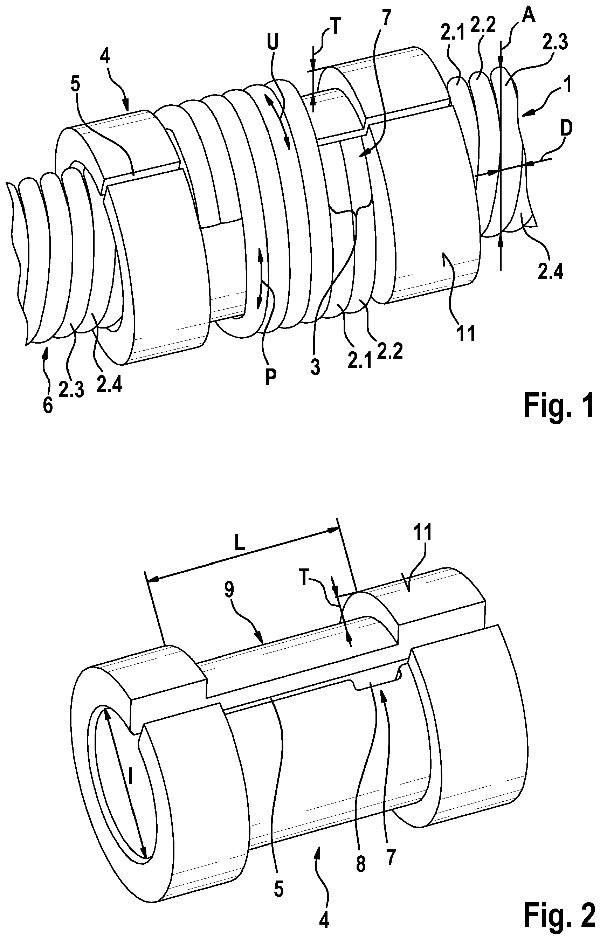

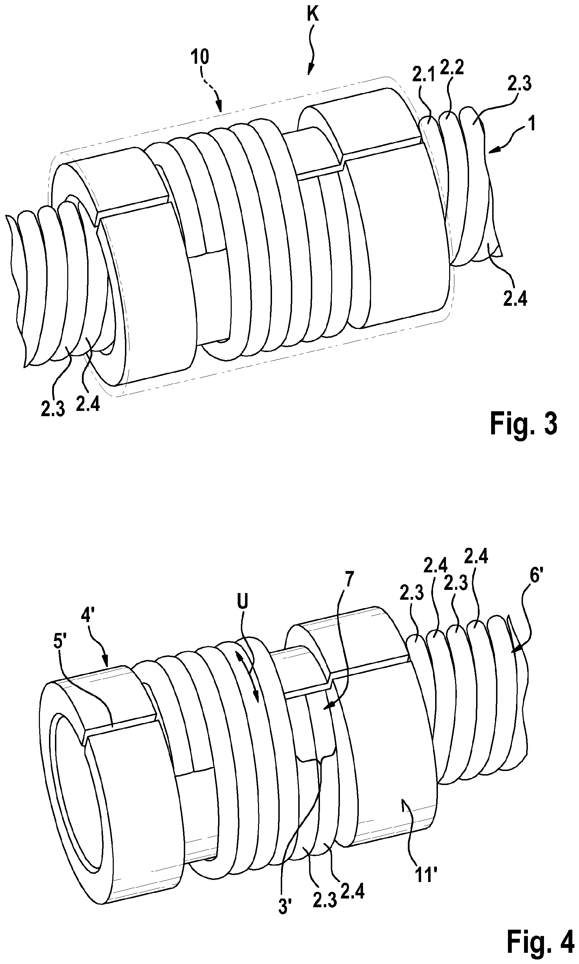

Wie in Fig. 1 und 3 erkennbar ist, wird die gezeigt Kontaktierungsvorrichtung K an einer Leitungswendel 1 mit vierfach, koradial gebündelten Wendeldrähten 2.1 - 2.4 verwendet. Letztere sind einzeln isoliert, wie in der Zeichnung nicht näher dargestellt ist. Im Bereich der Kontaktierungsvorrichtung K wird ein Teilbündel 3 aus zwei benachbarten Wendeldrähten 2.1, 2.2 vom Wendelverbund in einem nicht näher dargestellten Vormontagezustand tangential geradlinig weggeführt. Von diesem Teilbündel 3 wird die Isolationsschicht der einzelnen Wendeldrähte 2.1, 2.2 entfernt.

Für den in Fig. 1 gezeigten Fertigungszwischenschritt wird dann eine durch einen in axialer Richtung verlaufenden, durchgehenden Durchführungsschlitz 5 offene Fixier-Hülse 4 über die aus den beiden verbleibenden Wendeldrähten 2.3, 2.4 gebildete Restwendel 6 bis zur Sollposition der Kontaktierungsvorrichtung K bei dem herausgeführten Teilbündel 3 aufgeschoben. Das Teilbündel 3 wird durch den Durchführungsschlitz 5 geführt, wobei an der radialen Austrittsposition 7 im Durchführungsschlitz 5 eine Verbreiterung in Form einer Freimachung 8 (siehe Fig. 2) vorgesehen ist. Die Fixier-Hülse 4 ist dabei durch den - wie aus Fig. 3 erkennbaren - aufgedehnten Durchführungsschlitz 5 mit einem gegenüber dem Außendurchmesser A der Leitungswendel 1 größeren Innendurchmesser I versehen.

Zur Erzielung des in Fig. 1 gezeigten Fertigungszwischenschrittes wird dann die Fixier-Hülse 4 durch Zusammendrücken plastisch verformt, bis der Durchführungsschlitz 5 die in Fig. 1 gezeigte, enge Konfiguration einnimmt und das Teilbündel 3 im Bereich des Durchführungsschlitzes 5 fixiert ist. Anschließend wird das Teilbündel 3 unter radialer Spannung in eine in der Mantelfläche der Fixier-Hülse 4 angelegten, in Peripherrichtung P umlaufenden Wickelnut 9 auf Block gewickelt. Wenngleich in Fig. 1 ein Umschlingungswinkel U des Teilbündels 3 mehr als 3 x 360°, also mehr als 1080° gezeigt ist, reicht für eine sichere mechanische und elektrische Verbindung zwischen dem Teilbündel 3 und der Fixier-Hülse 4 ein relativ geringer Umschlingungswinkel U von 60° bis 70°.

Zur zusätzlichen mechanischen Sicherung des Teilbündels 3 auf der Fixierhülse 4 und Kontaktierung zwischen diesen beiden Bauteilen werden die Wendeldrähte 2.1, 2.2 des Teilbündels 3 an geeigneter Stelle in der Wickelnut 9 mit der Fixier-Hülse 4 verschweißt.

Wie aus Fig. 1 deutlich wird, entspricht die radiale Tiefe T der Wickelnut dem Durchmesser D der Wendeldrähte 2.1, 2.2. Ihre radiale Länge L liegt bei etwas mehr als dem achtfachem Durchmesser D der Wendeldrähte 2.1, 2.2.

Die Komplettierung der Kontaktierungseinrichtung K erfolgt - wie aus Fig. 3 hervorgeht - durch Aufschieben der ringförmigen Elektroden-Hülse 10, deren axiale Länge mit der der Fixier-Hülse 4 übereinstimmt. In der in Fig. 3 gezeigten Position wird die Elektroden-Hülse 10 mit der Fixier-Hülse 4 verschweißt, sodass die Elektroden-Hülse 10 zuverlässig elektrisch kontaktiert ist. Ihre Mantelfläche 11 bildet die Elektrodenfläche der so realisierten Ringelektrode an einer implantierbaren Elektrodenleitung, wie beispielsweise einer Herzschrittmacher-Elektrode.

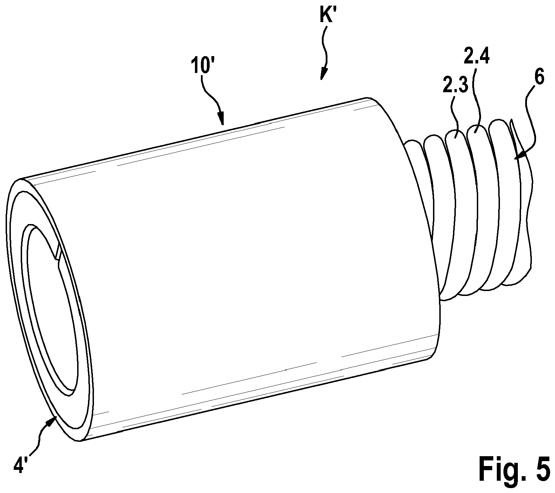

Die in Fig. 4 und 5 gezeigte Kontaktierungseinrichtung K' realisiert eine Kopfelektrode, wie sie beispielsweise an der aus den beiden Wendeldrähten 2.3, 2.4 gebildeten Restwendel 6' an deren distalem Ende angeordnet sein kann. Die Kontaktierungseinrichtung K' ist im Wesentlichen baugleich mit der Kontaktierungseinrichtung K, weist also in analoger Weise eine Fixier-Hülse 4' auf, die jedoch im Durchmesser erforderlichenfalls - sofern die Restwendel 6' im Durchmesser geringer als die Leitungswendel 1 ausgeführt ist - kleiner dimensioniert sein kann. Im Übrigen sind alle Komponenten der Kontaktierungseinrichtung K' mit den Komponenten der Kontaktierungseinrichtung K entsprechenden, allerdings apostrophierten Bezugsziffern versehen. Eine nochmalige Erörterung der Kontaktierungseinrichtung K' erübrigt sich demzufolge. Es ist lediglich zu wiederholen, dass die auf die Fixier-Hülse 4' nach dem Kontaktieren der Restewendel 6 aufgeschobene Elektroden-Hülse 10' mit ihrer Mantelfläche 11' die Kopfelektrode einer Elektrodenleitung, also beispielsweise die Elektrodenfläche einer Herzschrittmacher-Elektrode, bildet.

## Patentansprüche

1. Kontaktierungseinrichtung für elektrische Verbindungen an flexiblen, in einen Patientenkörper einführbaren, insbesondere implantierbaren Elektrodenleitungen, umfassend
- eine Leitungswendel (1) mit mehreren, koradial gebündelten Wendeldrähten (2.1 - 2.4),
- eine innere Fixier-Hülse (4, 4') für ein Teilbündel (3, 3') der Wendeldrähte (2.1, 2.2), wobei die Fixier-Hülse (4, 4') einen in axialer Richtung verlaufenden Durchführungsschlitz (5, 5') für das Teilbündel (3, 3') der Wendeldrähte (2.1 - 2.4), eine in Peripherrichtung (P) umlaufende Wickelnut (9, 9') für das herausgeführte Teilbündel (3, 3') sowie eine Freimachung (8) am Durchführungsschlitz (5, 5') zum Hindurchführen des Teilbündels (3, 3') aufweist,
- eine äußere, auf der inneren Fixierhülse (4, 4') sitzenden, mit dem Teilbündel (3, 3') elektrisch kontaktierte Elektroden-Hülse (10, 10'), und
- eine zugsichere Fixierung zwischen der Fixier-Hülse (4, 4') und dem durch die Freimachung (8) geführten Teilbündel (3, 3') durch eine Umschlingung des herausgeführten Teilbündels (3, 3') um die Fixier-Hülse (4, 4') in der Wickelnut (9, 9') um einen Mindest-Umschlingungswinkel (U),
**dadurch gekennzeichnet, dass**
der Durchführungsschlitz (5, 5') in axialer Richtung durchgehend in der Fixierhülse (4, 4') angelegt und zur zusätzlichen zugsicheren Fixierung zwischen der Fixier-Hülse (4, 4') und dem Teilbündel (3, 3') die Fixier-Hülse (4, 4') auf die Leitungswendel (1) unter plastischer Verformung aufgepresst ist.

2. Kontaktierungseinrichtung nach Anspruch 1, **dadurch gekennzeichnet, dass** die Leitungswendel (1) eine Vierfach- oder Achtfach-Koradial-Wendel ist.

3. Kontaktierungseinrichtung nach Anspruch 1 oder 2, **dadurch gekennzeichnet, dass** das Teilbündel (3, 3') zwei benachbarte Wendeldrähte (2.1, 2.2) umfasst.

4. Kontaktierungseinrichtung nach einem der vorgenannten Ansprüche, **dadurch gekennzeichnet, dass** zur Umschlingung des Teilbündels (3, 3') dessen mindestens ein Wendeldraht (2.1, 2.2) unter radialer Vorspannung in die Wickelnut (9, 9') auf der Fixier-Hülse (4, 4') gewickelt ist.

5. Kontaktierungseinrichtung nach einem der vorgenannten Ansprüche, **dadurch gekennzeichnet, dass** der Mindest-Umschlingungswinkel (U) des Teilbündels (3, 3') um die Fixier-Hülse (4, 4') mindestens 60° beträgt.

6. Kontaktierungseinrichtung nach einem der vorgenannten Ansprüche, **dadurch gekennzeichnet, dass** die Wendeldrähte (2.1 - 2.4) mit einer Isolierung versehen sind, die im Bereich des herausgeführten Teilbündels (3, 3') entfernt ist.

7. Kontaktierungseinrichtung nach einem der vorgenannten Ansprüche, **dadurch gekennzeichnet, dass** der mindestens eine Wendeldraht (2.1, 2.2) des herausgeführten Teilbündels (3, 3') mit der Fixier-Hülse (4, 4') in einem stoffschlüssigen Fügeverfahren miteinander elektrisch und mechanisch verbunden ist.

8. Kontaktierungseinrichtung nach Anspruch 7, **dadurch gekennzeichnet, dass** der mindestens eine Wendeldraht (2.1, 2.2) des herausgeführten Teilbündels (3, 3') mit der Fixier-Hülse (4, 4') miteinander verschweißt sind

9. Kontaktierungseinrichtung nach einem der Ansprüche 1 bis 6, **dadurch gekennzeichnet, dass** der mindestens eine Wendeldraht (2.1, 2.2) des herausgeführten Teilbündels (3, 3') mit der Fixier-Hülse (4, 4') vorzugsweise unter Verwendung eines elektrisch leitfähigen Klebstoffes verklebt ist.

10. Kontaktierungseinrichtung nach einem der vorgenannten Ansprüche, **dadurch gekennzeichnet, dass** für eine Kontaktierung einer Ringelektrode die nach dem Herausführen des Teilbündels (3) verbleibende Restwendel (6) über die Fixierhülse (4) hinaus weiter geführt und die Elektroden-Hülse (10) über die Restwendel (6) hinweg auf die Fixier-Hülse (4) aufgeschoben und dort fixiert ist.

11. Kontaktierungseinrichtung nach einem der Ansprüche 1 bis 9, **dadurch gekennzeichnet, dass** für eine Kontaktierung einer Kopfelektrode die nach dem Herausführen des Teilbündels (3') verbleibende Restwendel in der Fixierhülse (4') endet und die Elektroden-Hülse (10') auf die Fixier-Hülse (4') aufgeschoben und dort fixiert ist.

12. Kontaktierungseinrichtung nach einem der vorgenannten Ansprüche, **dadurch gekennzeichnet, dass** die radiale Tiefe (T) der Wickelnut (9, 9') mindestens dem Durchmesser (D) des Wendeldrahtes (2.1 - 2.4) entspricht.

13. Kontaktierungseinrichtung nach einem der vorgenannten Ansprüche, **dadurch gekennzeichnet, dass** die axiale Länge (L) der Wickelnut (9, 9') mindestens dem Durchmesser (D), vorzugsweise einem ganzzahligen Vielfachen des Durchmessers (D) des Wendeldrahtes (2.1 - 2.4) entspricht.

## Claims

1. A contacting device for electrical connections to flexible electrode lines insertable, in particular implantable, into a patient body, comprising
- a line coil (1) with a plurality of coradially bundled coil wires (2.1-2.4),
- an inner fixing sleeve (4, 4') for a partial bundle (3, 3') of the coil wires (2.1, 2.2), wherein the fixing sleeve (4, 4') has a through-slit (5, 5') running in the axial direction for the partial bundle (3, 3') of the coil wires (2.1-2.4), a winding groove (9, 9') running in the peripheral direction (P) for the partial bundle (3, 3') led through, and an exposure (8) at the through-slit (5, 5') in order to pass through the partial bundle (3, 3'),
- an outer electrode sleeve (10, 10') sitting on the inner fixing sleeve (4, 4') and electrically contacted with the partial bundle (3, 3'), and
- a strain-resistant fixing between the fixing sleeve (4, 4') and the partial bundle (3, 3') guided through the exposure (8), provided by looping the led-out partial bundle (3, 3') around the fixing sleeve (4, 4') in the winding groove (9, 9') by a minimum looping angle (U),
**characterised in that**
the through-slit (5, 5') is formed in the fixing sleeve (4, 4') continuously in the axial direction and, for additional strain-resistant fixing between the fixing sleeve (4, 4') and the partial bundle (3, 3'), the fixing sleeve (4, 4') is pressed onto the line coil (1) so as to produce plastic deformation.

2. The contacting device according to claim 1, **characterised in that** the line coil (1) is a multiple or eightfold coradial coil.

3. The contacting device according to claim 1 or 2, **characterised in that** the partial bundle (3, 3') comprises two adjacent coil wires (2.1, 2.2).

4. The contacting device according to any one of the preceding claims, **characterised in that,** to loop the partial bundle (3, 3'), at least one coil wire (2.1, 2.2) thereof is wound under radial bias into the winding groove (9, 9') on the fixing sleeve (4, 4').

5. The contacting device according to any one of the preceding claims, **characterised in that** the minimum looping angle (U) of the partial bundle (3, 3') around the fixing sleeve (4, 4') is at least 60°.

6. The contacting device according to any one of the preceding claims, **characterised in that** the coil wires (2.1-2.4) are provided with an insulation, which is removed in the region of the led-out partial bundle (3, 3').

7. The contacting device according to any one of the preceding claims, **characterised in that** the at least one coil wire (2.1, 2.2) of the led-out partial bundle (3, 3') is connected electrically and mechanically to the fixing sleeve (4, 4') in an integrally bonding joining method.

8. The contacting device according to claim 7, **characterised in that** the at least one coil wire (2.1, 2.2) of the led-out partial bundle (3, 3') is welded to the fixing sleeve (4, 4').

9. The contacting device according to any one of claims 1 to 6, **characterised in that** the at least one coil wire (2.1, 2.2) of the led-out partial bundle (3, 3') is adhesively bonded to the fixing sleeve (4, 4'), preferably with use of an electrically conductive adhesive.

10. The contacting device according to any one of the preceding claims, **characterised in that,** for contacting of a ring electrode, the rest of the coil (6) remaining once the partial bundle (3) has been led out is guided further beyond the fixing sleeve (4), and the electrode sleeve (10) is slid onto the fixing sleeve (4) over the rest of the coil (6) and is fixed there.

11. The contacting device according to any one of claims 1 to 9, **characterised in that,** for contacting of a head electrode, the rest of the coil remaining once the partial bundle (3') has been led out ends in the fixing sleeve (4'), and the electrode sleeve (10') is slid onto the fixing sleeve (4') and is fixed there.

12. The contacting device according to any one of the preceding claims, **characterised in that** the radial depth (T) of the winding groove (9, 9') corresponds at least to the diameter (D) of the coil wire (2.1-2.4).

13. The contacting device according to any one of the preceding claims, **characterised in that** the axial length (L) of the winding groove (9, 9') corresponds at least to the diameter (D), preferably to an integer multiple of the diameter (D), of the coil wire (2.1-2.4).

## Revendications

1. Dispositif de contact pour des connexions électriques à des lignes d'électrodes souples, pouvant être insérées, notamment, pouvant être implantées dans le corps d'un patient, comprenant
- une bobine de ligne (1) avec plusieurs fils hélicoïdaux (2.1 à 2.4) coaxiaux groupés en faisceaux,
- une cosse de fixation interne (4, 4') pour un faisceau partiel (3, 3') des fils hélicoïdaux (2.1, 2.2), où la cosse de fixation (4, 4') présente une fente de passage (5, 5'), s'étendant dans la direction axiale, pour le faisceau partiel (3, 3') des fils hélicoïdaux (2.1 à 2.4), une rainure d'enroulement (9, 9') parcourant le pourtour dans la direction périphérique (P), pour le faisceau partiel (3, 3') mené vers l'extérieur, ainsi qu'un système de libération (8) sur la fente de passage (5, 5') pour faire passer le faisceau partiel (3, 3') à travers,
- une cosse d'électrode (10, 10') externe, logée sur la cosse de fixation interne (4, 4'), étant en contact électrique avec le faisceau partiel (3, 3'), et
- une fixation résistant à une traction entre la cosse de fixation (4, 4') et le faisceau partiel (3, 3') mené à travers le système de libération (8) par le biais d'un enlacement du faisceau partiel (3, 3') mené vers l'extérieur autour de la cosse de fixation (4, 4') dans la rainure d'enroulement (9, 9') équivalent à un angle d'enlacement minimal (U),
**caractérisé en ce que**
la fente de passage (5, 5') est agencée en direction axiale, traversant totalement la cosse de fixation (4, 4'), et la cosse de fixation (4, 4') est pressée sur la bobine de ligne (1), moyennant une déformation plastique, pour la fixation complémentaire résistant à une traction entre la cosse de fixation (4, 4') et le faisceau partiel (3, 3').

2. Dispositif de contact selon la revendication 1, **caractérisé en ce que** la bobine de ligne (1) est une bobine à quatre fils ou à huit fils coaxiaux.

3. Dispositif de contact selon la revendication 1 ou 2, **caractérisé en ce que** le faisceau partiel (3, 3') comprend deux fils hélicoïdaux (2.1, 2.2) voisins.

4. Dispositif de contact selon l'une des revendications précitées, **caractérisé en ce que,** pour l'enlacement du faisceau partiel (3, 3'), son au moins un fil hélicoïdal (2.1, 2.2) est enroulé sur la cosse de fixation (4, 4') dans la rainure d'enroulement (9, 9') moyennant une précontrainte radiale.

5. Dispositif de contact selon l'une des revendications précitées, **caractérisé en ce que** l'angle d'enlacement minimal (U) du faisceau partiel (3, 3') autour de la cosse de fixation (4, 4') est au moins égal à 60 °.

6. Dispositif de contact selon l'une des revendications précitées, **caractérisé en ce que** les fils hélicoïdaux (2.1 à 2.4) sont munis d'une isolation qui est enlevée dans la région du faisceau partiel (3, 3') menée vers l'extérieur.

7. Dispositif de contact selon l'une des revendications précitées, **caractérisé en ce que** l'au moins un fil hélicoïdal (2.1, 2.2) du faisceau partiel (3, 3') mené vers l'extérieur est relié électriquement et mécaniquement avec la cosse de fixation (4, 4') dans un procédé de liaison par complémentarité de matière.

8. Dispositif de contact selon la revendication 7, **caractérisé en ce que** l'u moins un fil hélicoïdal (2.1, 2.2) du faisceau partiel (3, 3') mené vers l'extérieur est soudé ensemble avec la cosse de fixation (4, 4').

9. Dispositif de contact selon l'une des revendications 1 à 6, **caractérisé en ce que** l'au moins un fil hélicoïdal (2.1, 2.2) du faisceau partiel (3, 3') est collé avec la cosse de fixation (4, 4'), de préférence moyennant l'emploi d'un adhésif électriquement conducteur.

10. Dispositif de contact selon l'une des revendications précitées, **caractérisé en ce que,** pour la mise en contact d'une électrode annulaire, la bobine résiduelle (6) restant après l'extraction du faisceau partiel (3) est menée encore plus vers l'extérieur par-dessus la cosse de fixation (4) et la cosse d'électrode (10) est poussée au-delà sur la cosse de fixation (4) par-dessus la bobine résiduelle (6) et est fixée à cet endroit.

11. Dispositif de contact selon l'une des revendications 1 à 9, **caractérisé en ce que** pour une mise en contact d'une électrode frontale, la bobine résiduelle restant après l'extraction du faisceau partiel (3') s'arrête dans la cosse de fixation (4') et la cosse d'électrode (10') est poussée sur la cosse de fixation (4') et est fixée à cet endroit.

12. Dispositif de contact selon l'une des revendications précitées, **caractérisé en ce que** la profondeur radiale (T) de la rainure d'enroulement (9, 9') correspond au moins au diamètre (D) du fil hélicoïdal (2.1 à 2.4).

13. Dispositif de contact selon l'une des revendications précitées, **caractérisé en ce que** la longueur axiale (L) de la rainure d'enroulement (9, 9') correspond au moins au diamètre (D), de préférence à un multiple entier du diamètre (D), du fil hélicoïdal (2.1 à 2.4).
